# EUROPEAN PATENT APPLICATION

(11) **EP 0 599 766 A1**
(43) Date of publication of application: **01.06.1994**
(21) Application number: 93500127.1
(22) Date of filing: 03.09.1993
(51) Int. Cl.: A61B 17/56

(54) **Cervical vertebral fusion system**

(30) Priority: 07.09.1992 ES 9201814; 01.09.1993 ES 9301886
(71) Applicant: Barbera Alacreu, José Vicente, E-46009 Valencia (ES)
(72) Inventor: Barbera Alacreu, José Vicente, E-46009 Valencia (ES)
(74) Representative: Flaccus, Rolf-Dieter, Dr.

(57) **Abstract**

The system is applicable in surgical procedures involving the cervical spine with anterior approach, specifically in surgery of the intervertebral disc involving disc removal and the subsequent application of a graft (1) in a cavity defined between the corresponding vertebrae (5) to achieve the corresponding bone consolidation. In this system, the graft (1) is immobilised by a plate (4) secured to the front of the vertebrae (5) by expandable screws (2) passing through this plate (4) and anchored to the vertebrae (5) which contain one or more screws (3) to anchor the corresponding graft (1). The expansion screws (2) consist of an outer anchoring screw with cross slits in its distal point and an inner threaded screw in its interior which, when inserted, causes the outer anchoring screw to enlarge and thus anchors the entire assembly to the vertebrae (5).

## Description

### FIELD OF THE INVENTION

This invention, as expressed by the title of this descriptive report, refers to a system of cervical vertebral fusion which involves surgical treatment by the anterior approach to treat cervical spine pathologies based on the implantation of a graft in the intervertebral space resulting from disc removal. The system utilizes a plate and expandable screws to immobilise the graft and attain the highest guarantee of good bone consolidation.

### BACKGROUND OF THE INVENTION

As is known, cervical spine surgery is indicated in multiple processes, including traumatic, degenerative, inflammatory, tumoral and congenital pathologies although all these pathologies have different characteristics from a therapeutical point of view depending on the vertebral level affected. Basically, two levels can be identified, one defined as the high cervical spine which includes the first and second cervical vertebrae, and the other denominated the lower cervical spine which includes the 3rd-7th vertebral segments. Injuries at these levels are much more frequent and require specific surgical treatment more often.

In this regard, cervical spine pathologies can be approached surgically from two directions: the posterior approach which requires performing a laminectomy and the anterior approach which involves access through the anterior or anterolateral aspect of the neck. The latter is more widely used since the pathology has a more frequently anterior location (in the vertebrae themselves or the intervertebral discs).

This approach to the spine was well systematised by Cloward (1958) and by Robinson and Smith (1958). A vertical or transverse incision, located on the anterior edge of the sternocleidomastoid muscle, is made to access the prevertebral space between the vascularcarotid-jugular bundle on the outside and the pharynx-larynx on the inside.

The anterior common longitudinal ligament is removed, exposing the discs and vertebrae at one or more levels.

The next step depends on the type of pathology to be treated (somatic or discal vertebral), but in any case, a general repercussion is a uni- or multi-segment instability caused by the pathology itself, the ligament interruption or the removal of the disc or portions of the vertebrae.

In conclusion, cervical spine instability is the most frequent side-effect of the actual surgical procedure through the anterior approach. Precisely for this reason, since surgery by the anterior approach began to be performed, procedures were designed to ensure post-operative stability of the segment operated on.

Intervertebral disc surgery occurs most frequently and, as a result of this, stabilization techniques have been developed.

In 1958 Cloward proposed the placement of an intersomatic graft in the intervertebral space produced upon disc removal. After removing the disc with a drill specially designed for the purpose, a cylindrical cavity extending to the body of the superior and inferior vertebrae is created in the intervertebral space. Afterwards, another drill of similar diameter is used to obtain a cylinder of spongy bone tissue from the ala of the ilium of the patient. This cylinder is introduced into the cavity created in the intervertebral space in order to attain bone fusion. Recently Otero modified the technique in such a way that the bone cylinder and the intervertebral cavity are threaded so as to facilitate graft placement.

Cloward's technique has been and is still being widely utilized. It achieves good bone consolidation in the space of two or three months. Its disadvantages are the obtaining of the graft and the risk of perforating the posterior vertebral wall and thus producing a medullar injury.

In 1958 Smith and Robinson published their technique, which is similar to that of Cloward. The intervertebral space produced upon disc removal is filled with a bone graft taken from the iliac crest. The procedure is technically simpler than Cloward's but has the disadvantage that the placement of the graft in its new location is weaker. Extension movements of the cervical spine can cause the bone to be expelled forward.

Verbiest, to avoid this latter problem, proposes carving the vertebral platforms after removing the disc in such a way that they form a wedge-shaped cavity, with an anterior base. The graft is also cut in the shape of a wedge. By producing a forced extension of the cervical spine, it is possible to position the graft in its cavity, but this requires very meticulous and long surgery.

To avoid the problems of graft expulsion by ensuring cervical spine immobility during the consolidation process, in 1972, Orozco and Llobet began to use small screwed plates anchored in the adjacent vertebral bodies. There are many models and types of plates and screws. Practically all of them have the same disadvantage. The high degree of mobility of the cervical spine causes the osteosynthesis material to be expelled. To avoid this, the anchoring screws must cross the posterior cortical layer of the vertebral body, with the added risk of medullar lesion.

The most recent technique is that reported by Caspar. This author utilizes large plates, about 15 or 20 cm. wide, which cover the anterior face of the vertebral bodies and of the removed disc. The plates are anchored to the vertebrae by several screws, two for each level. The solidity obtained is greater but the surgery required is more aggressive and longer and there is still a risk of medullar injury produced by the screws, which must perforate the posterior cortical layer of the vertebra. The most recent contribution to this technique is that of Synthes screws, which expand at the head level when another small central screw is introduced into them. This provides greater solidity of the screw-plate bond with a reduction in mobilisation capacity.

Following this brief review of the currently used techniques, it is possible to conclude that the common disadvantages to all the procedures are:
- Risk of medullar injury produced by the need to perforate the posterior wall of the vertebral body to anchor the screws.
- Meticulousness of the technique, due to the previously mentioned risk, which requires the prolonged usage of a radiological imaging intensifier and its inherent risks for the surgeon.
- Frequency with which mobilisation of the osteosynthesis material occurs with the risk of expulsion and requirement for additional surgery due to the high degree of mobility of the cervical spine.
- Necessity of prolonged immobilisation through use of a post-operative collar to avoid complications arising from the expulsion of the material.
- Aggressiveness of the surgery, with the need to expose many fields, to separate the muscles from the anterior spine and to take too much time. These characteristics are all valid for Caspar's technique which is the most widely used today.

All of these disadvantages could be overcome with a vertebral fusion system which would ensure a solid anchoring of the plate to the vertebrae.

### DESCRIPTION OF THE INVENTION

The field of the invention consists of a system by which vertebral fusion is attained with a complete guarantee of a solid anchoring of the plate to the vertebrae through the use of expandable screws together with the plate itself.

For this purpose, the system includes a plate set on the anterior face of the vertebrae, the screws which pass through the plate and are then attached to the vertebrae itself, and the screw or screws which also pass through the plate and are attached to the graft. The anchoring screws for the vertebrae are cylindrical, preferably 12 mm. long and 7-8 mm. in diameter, have a wide thread and a blunt point and are self-tapping. These screws are called anchoring screws because another small screw is placed within their centre. For this purpose, the anchoring screw features longitudinal slits about 4-5 mm. long and is hollow and threaded on the inside so as to hold the inner screw. This inner screw opens the anchoring screw's points as it enters and increases its diameter when the inner screw is in place. This increase is 25-30% with respect to the original diameter and provides a solid anchoring to the vertebra, making it impossible to be expelled.

Logically, the screws mentioned pass through the bonding plate which is approximately 10 mm. wide and of varying lengths depending on the number of levels which must be fused and have orifices with a special cavity to accommodate the head of the anchoring screw so that the bond between the anchoring screw and the plate is completely solid.

The screw or screws which pass through the plate and anchor the graft are conventional screws with a head which stays positioned in the previously mentioned recess in the corresponding plate. These screws consist solely of a simple shank with an appropriate thread and its respective head.

Both the vertebra anchoring screws and the graft anchoring screws as well as the plates themselves are made of titanium to ensure the possibility of post-operative exploration through magnetic resonance imaging. The anchoring screw's external surface, including its spiral or thread, is coated with hydroxyapatite to facilitate the implant's solidity and encourage bone growth around it.

Based on these structural and application characteristics, the advantages offered by this system of expandable screws can summarized as follows:
- Solidity of the system which hinders the expulsion of the osteosynthesis, through the distal? dilation of the anchoring screw.
- The risks of perforating the posterior cortical layer of the vertebral body are avoided since this perforation is no longer necessary.
- The irradiation time for the surgeon is reduced since radiological monitoring is practically unnecessary since the anchoring screws are quite short and can never perforate the posterior wall.
- The severity of the surgery itself is reduced since the material to be implanted does not require large incisions or prevertebral muscular detachment.
- The need for a collar or any other form of post-operative immobilisation becomes unnecessary, due to the assembly's solidity.
- Fusion is ensured even in patients with osteoporotic bones.

To supplement the description provided below and to aid in a better understanding of the characteristics of the invention, a set of diagrams are attached to this descriptive report. These drawings allow a better understanding of the innovations and advantages of the cervical vertebral fusion system in accordance with the field of the invention.

### BRIEF DESCRIPTION OF THE DRAWINGS

Figure 1 provides a schematic representation of a section, showing a vertical lateral view with the system implemented according to the field of the invention.

Figure 2 provides a schematic front view of the same applied system.

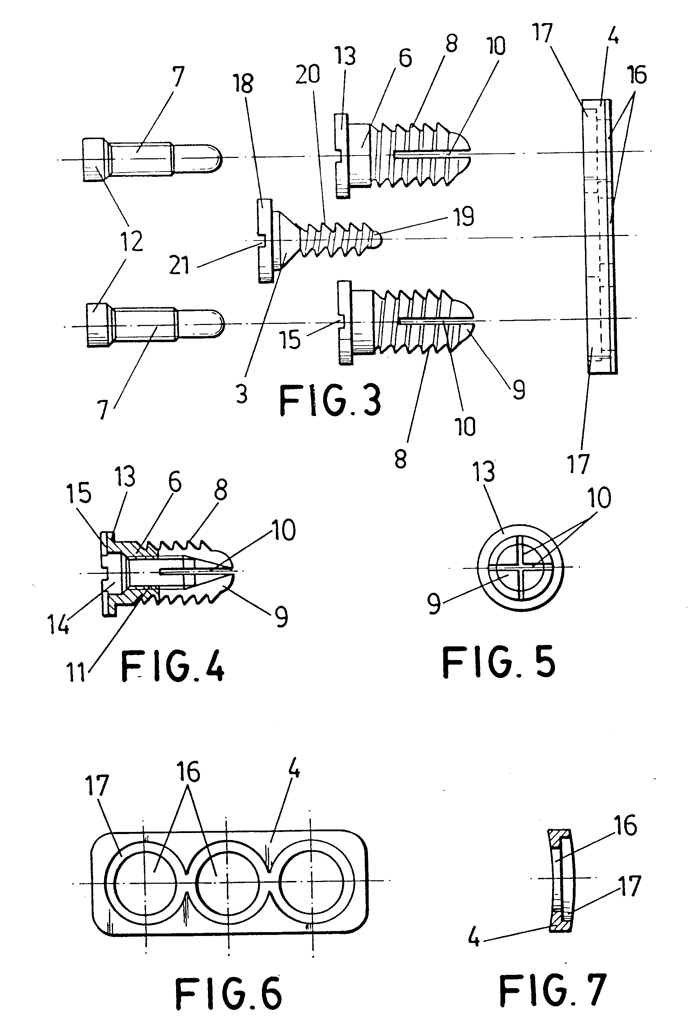

Figure 3 provides a lateral view amplifying the different elements making up the invention's system.

Figure 4 provides a longitudinal view of the expandable screw.

Figure 5 provides a view of the distal point of the anchoring screw shown in the previous figure in which the slits in form of a cross can be seen.

Figure 6 provides a view of the plate that belongs to the invention's system, in one of its possible configurations.

Figure 7 provides a cross-sectional view of the plate shown in the previous figure.

Figure 8 is a side view of the expandable screw of one of its possible variations.

Figure 9 corresponds to a cross-sectional view of the plate with two expandable screws, of the type shown in Figure 8, inserted at divergent angles.

Figure 10 shows the plate with two orifices and a central orifice with a smaller radius in one of its possible configurations.

Figure 11 is a cross-sectional view of the plate at A-A'.

Figure 12 shows the longitudinal profile of the non-curvable plate to match the anterior profile of the cervical spine.

### DESCRIPTION OF THE PREFERRED APPLICATION

In view of the mentioned figures, it is possible to observe how the invention's system envisions surgical application to cervical spine pathologies through the anterior approach and is specifically applicable to intervertebral disc surgeries, so that once the disc is removed, the corresponding intervertebral cavity created as a result contains a graft (1). This graft is immobilised precisely by the invention's system which consists of screws (2) and (3) and plate (4) in such a way that the screws (2) are anchored to the corresponding vertebrae (5) whilst the screw or screws (3) are anchored to the graft itself (1). This secures the plate to the anterior face of the vertebrae (5) precisely by means of these screws.

Screw (2) consists of what could be considered an anchoring screw (6) and an inner screw (7), both made of the same material, which is titanium. The anchoring screw (6) is hollow and contains an outer spiral (8) consisting of a thread, a distal blunt point (9) and longitudinal slits in the form of a cross (10) with the unique feature that the inside of this anchoring screw (6) also contains a thread or spiral (11) to match the thread of the inner screw (7). This inner screw has a head (12) permitting the use of an Allen wrench, screwdriver or other appropriate tool. The anchoring screw (6) also has the unique feature that its head (13) has a cavity (14) designed to hold the head (12) of screw (7) and also has several slots (15) to allow it to be handled by an appropriate tool or similar instrument.

On the other hand, the plate (4) is also made of titanium and has a flat-rectangular configuration, with orifices (16) to permit the passage of screws (2) and (3) which are indented around their perimeter (17) so as to accommodate the respective heads (13) of the screws (2) and for the heads (18) of the screws (3) utilized for the graft. These latter screws consist of a simple shank with a distal rounded point (19), a thread or external spiral (20) and head (18), also with the necessary slots (21) to permit handling with an appropriate tool. These screws (3) are made of titanium just as are the elements previously mentioned.

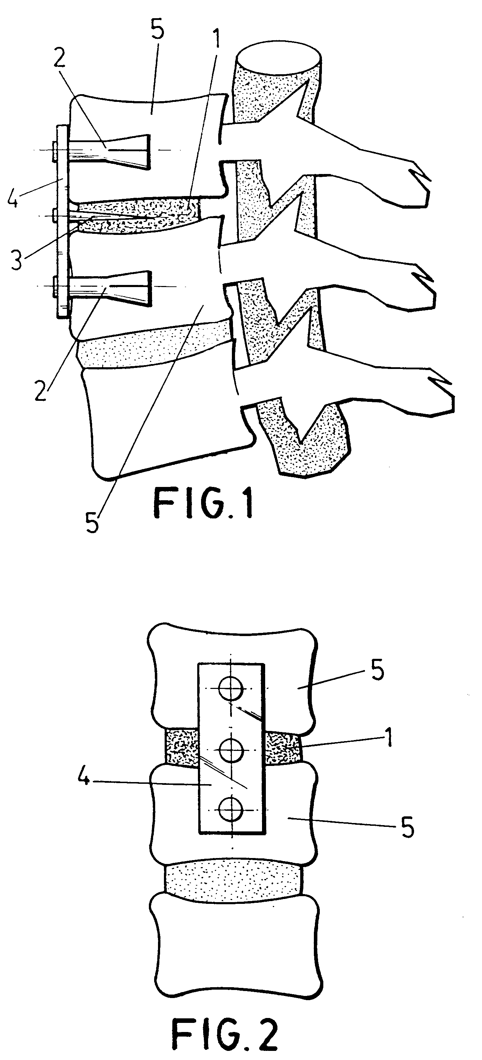

Thus, by using these elements and the system which they make up, the anchoring screws (6) are introduced first into the corresponding vertebrae (5), after passing through the plate (4), then the inner screw (7) is inserted into each anchoring screw (6). As this inner screw is advanced, it causes the distal point of the anchoring screw (6) to expand just as is presented clearly in the schematic diagram in Figure 1. This provides a secure anchoring of these screws (2) to the vertebrae (5) and thus, the immobilization of the plate (4), logically hindering the mobilization and expulsion of the graft (1). Screw (3) aids in this process.

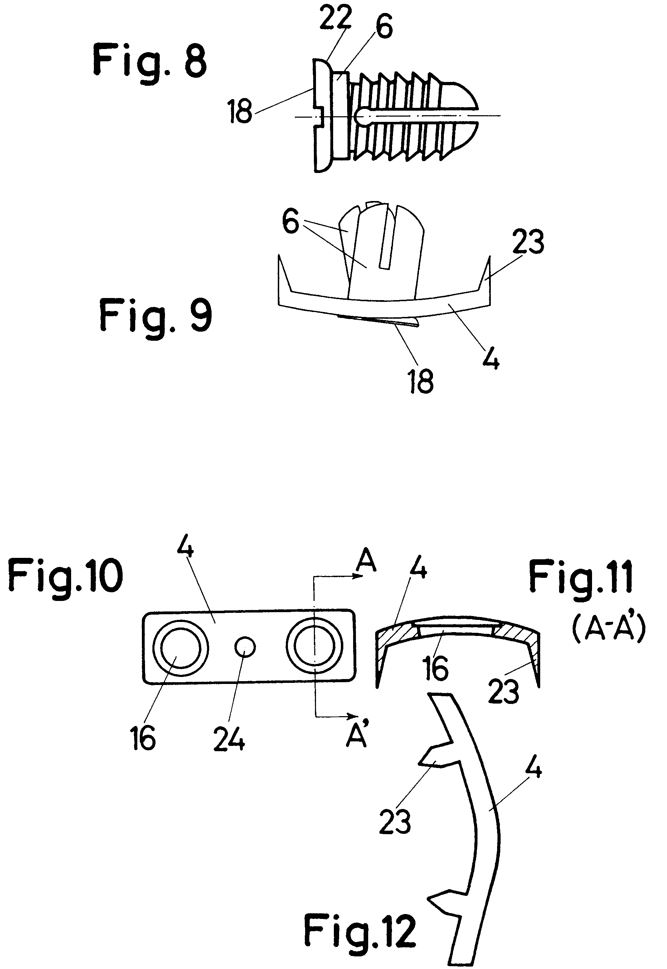

The anchoring screws (6) shown in Figures 8 and 9, have the advantage that their heads (18) are curved (22) to permit the angle of insertion into the hole (16) of the plate (4) to be varied and not necessarily be perpendicular. Thus, each screw can be inserted in a different direction, reinforcing the stability of the axial and lateral rotation of the vertebrae.

Lastly, it is possible to mention that plate (4), which has been shown in one of its possible applications with three orifices (16) can contain more orifices and even be wider or narrower. Thus, as shown in Figures 10, 11 and 12, the plates for two vertebral levels will have two large orifices (16) and a central orifice (24) of smaller radius. As a result, the plate (4) is more solid and weak points are eliminated.

The two-level plates will be available in various sizes and with lengths of 23, 27, 31 and 34 millimetres to accommodate the size of each patient's vertebrae.

The three-level plates will be manufactured in three lengths, the current one and another three millimetres longer.

In addition, the plates (4) have a three-millimetre prong (23) emerging perpendicularly from its posterior face - the one applied to the vertebra - and at the same horizontal level as the orifices (16) for the expandable screw. These prongs are located on each side and are embedded into the vertebra's anterior face, thus preventing shifts during lateral flexion and axial rotation.

## Claims

**1.-** A cervical vertebral fusion system, applicable in surgical procedures affecting the cervical spine performed through the anterior approach which involve the removal of the corresponding intervertebral disc and designed to ensure post-operative stability of the segment operated on. After the removal of the aforementioned intervertebral disc and the subsequent insertion of a graft (1) between the two vertebrae (5), the graft (1) is to be immobilised by means of several screws passing through the front plate which is secured to these vertebrae (5) through the aforementioned screws anchored to the vertebrae themselves (5), **wherein** these screws (2) for anchorage to the corresponding vertebrae (5) are short and consist of a hollow anchoring screw (6) featuring: an external spiral or thread (8) to anchor it to the respective vertebra (5), a rounded distal point (9), some longitudinal slits in the form of a cross (10) in the distal point and an internal spiral or thread (11) to hold the inner screw (7) which, as it enters, causes the anchoring screw (6) to expand and anchors it to the vertebrae (5).

**2.-** The cervical vertebral fusion system of claim 1, wherein the anchoring screw (6) features a head (13) with a matching internal recess (14) to hold the head (12) belonging to the inner screw (7) and whose head (13) contains slots (15) to permit the anchoring screw (6) to be handled by an appropriate tool. In addition, the inner screw (7) also has slots in its head to permit it to be handled with a tool.

**3.-** The cervical vertebral fusion system of any one of the foregoing claims, wherein the plate (4), which is secured to the front of the vertebrae (5) through screws (2) and through screw or screws (3) in which these latter screws serve to anchor the graft (1), contains the corresponding orifices (16) for these screws to pass and also a recess in its perimeter (17) to hold the respective heads (13) of the anchoring screw (6) corresponding to screw (2) and screws (3).

**4.-** The cervical vertebral fusion system of any one of the foregoing claims, wherein the length of the slots (10) of the anchoring screw (6) extend approximately three-fourths of the penetration length of the anchoring screw into the corresponding vertebra (5). The total length of this anchoring screw, and thus of screw (2) with the internal screw (3), is considerably shorter than the width of the vertebra (5) into which it is embedded.

**5.** The cervical vertebral fusion system of any one of the foregoing claims, wherein both the anchoring screws (6) and the inner screws (7) and graft screws (3) and the plate itself (4) are manufactured from a titanium material, and have the unique feature that the external surface of the anchoring screw (6) with its spiral or threading (8) is coated with hydroxyapatite.

**6.** The cervical vertebral fusion system of any of the foregoing claims, wherein the head (18) of the screws or expandable anchoring screws (6) have a curved profile (22) in the portion preceding the shank, to allow the insertion angle from the orifice (16) of plate (4) to be varied so that the screws can take different directions in order to reinforce axial and lateral rotational stability of the vertebrae.

**7.** The cervical vertebral fusion system of any of the foregoing claims, wherein the plates (4) have prongs (23) which emerge perpendicularly from the posterior side -or side applied to the vertebra surface- and whose prongs are located at the same height as that of the orifices (16) used for the expandable screws (6) and on each side of these screws. These prongs (23) are embedded into the anterior face of the vertebra to avoid shifts during lateral flexion and axial rotation.

**8.-** The cervical vertebral fusion system of any of the foregoing claims, wherein the plates (4) used for two vertebral levels contain two large orifices (16) and a central orifice (24) of smaller radius to provide greater consistency for these plates (4).
